# EUROPÄISCHE PATENTSCHRIFT

(11) **EP 1 900 392 B1**
(45) Veröffentlichungstag und Bekanntmachung des Hinweises auf die Patenterteilung: **03.11.2010**
(21) Anmeldenummer: 07114400.0
(22) Anmeldetag: 16.08.2007
(51) Int. Cl.: A61N 5/10

(54) **Strahlentherapieanlage, Verfahren zur Anpassung eines Bestrahlungsfeldes für einen Bestrahlungsvorgang eines zu bestrahlenden Zielvolumens eines Patienten**
Radiotherapy facility, method for adapting the radiation field for radiation treatment of the exposed body surface of the patient
Installation de radiothérapie, procédé destiné à l'adaptation d'un champ de rayonnement pour un processus de rayonnement d'un volume cible à rayonner sur un patient

(30) Priorität: 15.09.2006 DE 102006044139
(43) Veröffentlichungstag der Anmeldung: 19.03.2008
(73) Patentinhaber: SIEMENS AKTIENGESELLSCHAFT, 80333 München (DE)
(72) Erfinder: Rietzel, Eike, 64289 Darmstadt (DE)

(56) Entgegenhaltungen:
- EP-A- 0 986 070
- WO-A-03/076003
- JAEKEL O ET AL: "TREATMENT PLANNING FOR HEAVY ION IRRADIATION" PHYSICA MEDICA, ACTA MEDICA EDIZIONI E CONGRESSI, ROME, IT, Bd. 14, Nr. 1, 1997, Seiten 53-62, XP000908768 ISSN: 1120-1797
- KAMADA T ET AL: "A horizontal CT system dedicated to heavy-ion beam treatment" RADIOTHERAPY AND ONCOLOGY, ELSEVIER, Bd. 50, Nr. 2, Februar 1999 (1999-02), Seiten 235-237, XP002359765 ISSN: 0167-8140
- RIETZEL E ET AL: "Four-dimensional image-based treatment planning: Target volume segmentation and dose calculation in the presence of respiratory motion" INTERNATIONAL JOURNAL OF RADIATION: ONCOLOGY BIOLOGY PHYSICS, PERGAMON PRESS, US, Bd. 61, Nr. 5, 1. April 2005 (2005-04-01), Seiten 1535-1550, XP004842268 ISSN: 0360-3016
- PEDRONI E ET AL: "THE 200-MEV PROTON THERAPY PROJECT AT THE PAUL SCHERRER INSTITUTE: CONCEPTUAL DESIGN AND PRACTICAL REALIZATION" MEDICAL PHYSICS, AIP, MELVILLE, NY, US, Bd. 22, Nr. 1, Januar 1995 (1995-01), Seiten 37-53, XP000505145 ISSN: 0094-2405

## Beschreibung

Die Erfindung betrifft ein Strahlentherapieanlage zum Bestrahlen eines zu bestrahlenden Zielvolumens eines Patienten, wobei das Zielvolumen seine Lage und/oder oder Form im Patienten mit der Zeit verändern kann, mit einer Strahlenquelle, bei der ein applizierbares Bestrahlungsfeld einstellbar ist. Ferner betrifft die Erfindung ein Verfahren zur Anpassung eines Bestrahlungsfeldes für einen Bestrahlungsvorgang eines zu bestrahlenden Zielvolumens eines Patienten, wobei das Zielvolumen seine Lage und/oder Form im Patienten mit der Zeit verändern kann.

Strahlentherapie ist sowohl mit hochenergetischen Photonen als auch mit Partikeln möglich. Ersteres ist eine anerkannte Technik, wie sie z.B. aus US 6,687,330 B2 bekannt ist. Eine Partikeltherapieanlage weist üblicherweise eine Beschleunigereinheit und ein Hochenergiestrahlführungssystem auf. Die Beschleunigung der Partikel, z.B. Protonen, Kohlenstoff- oder Sauerstoffionen, erfolgt beispielsweise mit Hilfe eines Synchrotrons oder Zyklotrons.

Das Hochenergiestrahltransportsystem führt die Partikel von der Beschleunigereinheit zu einem oder mehreren Behandlungsräumen. Man unterscheidet zwischen "fixed beam"-Behandlungsräumen, in denen die Partikel aus einer festen Richtung auf den Behandlungsplatz treffen, und so genannten Gantry-basierten Behandlungsräumen. Bei letzteren ist es möglich, den Partikelstrahl mittels einer Gantry aus verschiedenen Richtungen auf den Patienten zu richten.

Ferner unterscheidet man zwischen so genannten Scanning- und Scattering-Techniken. Während letztere einen großflächigen auf die Ausmaße des zu bestrahlenden Volumens angepassten Strahl verwendet, wird bei der Scanning-Technik ein so genannter "pencil beam" von wenigen Millimetern bis Zentimetern Durchmesser über das zu bestrahlende Volumen gescannt. Bei der Ausführung eines Scan-Systems als Rasterscan-System wird der Partikelstrahl "punktweise" solange auf ein Volumenelement eines Rasters gelenkt, bis eine zuvor definierte Partikelzahl appliziert ist. Es werden alle Volumenelemente im Scanbereich nacheinander bestrahlt, wobei vorzugsweise die Ausdehnung der von nebeneinander applizierten Pencil Beams überlappt. Die Partikel für ein Volumenelement tragen nicht nur in diesem Volumenelement zur Dosis bei, sondern auch entlang des gesamten Partikeleinfallweges.

Ein Kontroll- und Sicherheitssystem der Partikeltherapieanlage gewährleistet, dass jeweils ein mit den erbetenen Parametern charakterisierter Partikelstrahl in den entsprechenden Behandlungsraum geleitet wird. Die Parameter eines Bestrahlungsvorgangs werden z.B. in einem so genannten Behandlungs- oder Therapieplan als Bestrahlungsfeld zusammengefasst. Dieser enthält eine Zuordnung von Teilchen zu Volumenelement, d.h., in ihm ist festgelegt, wie viele Teilchen aus welcher Richtung mit welcher Energie auf den Patienten bzw. die Volumenelemente treffen sollen. Die Energie der Partikel bestimmt deren Eindringtiefe in den Patienten, d.h. den Ort, an dem das Maximum der Wechselwirkung mit dem Gewebe bei der Partikeltherapie erfolgt; in anderen Worten, den Ort, an dem das Maximum der Dosis deponiert wird. Während der Behandlung befindet sich das Maximum der deponierten Dosis innerhalb des Tumors (oder im Fall von anderen medizinischen Anwendungen des Partikelstrahls im jeweiligen Zielgebiet).

Des Weiteren steuert das Kontroll- und Sicherheitssystem eine Positioniervorrichtung, mit der der Patient in Bezug zum Partikelstrahl positioniert wird. Für die Umsetzung des Therapieplans ist es wesentlich, dass der Patient für die Bestrahlung eine mit der Planung übereinstimmende Position einnimmt. Dies erfolgt z.B. mittels einer 2D-Positionsverifikation, bei der z.B. vor dem Durchführen der Bestrahlung ein Abgleich von 2D-Aufnahmen mit Aufnahmen aus der Bestrahlungsplanung erfolgt.

Eine Partikeltherapieanlagen mit einem Scanning-System ist z.B. aus EP 0 986 070 oder aus "The 200-MeV proton therapy project at the Paul Scherrer Institute: Conceptual design und practical realization", E. Pedroni et al., Med. Phys. 22, 37-53 (1995) bekannt.

Das Dokument "Treatment planning for heavy ion irradiation"; O. Jäkel und M. Krämer (Physica Medica- Vol. XIV, Supplement 1, July 1998), offenbart ein Verfahren zur Bestrahlungsplannung eines 3D-Plannungsbilddatensatzes für einen Bestrahlungsvorgang eines zu bestrahlendes Volumens eines Patienten, das mit einem Partikelstrahl mit Hilfe der Scanning Technik bestrahlt wird, bei der ein Partikelstrahl über das Zielvolumen punktweise gescannt wird wobei, zur Überwachung der Patientenpositionierung, ein Vergleichen eines in der Bestrahlungsphase gewonnenen Röntgenbildes mit einem DRR (Digitally Reconstructed Radiograph) Bild vorgesehen ist.

Im Folgenden wird die Erfindung vorwiegend im Zusammenhang mit einer Partikeltherapieanlage diskutiert, wobei sich die gleiche Problemstellung und Lösung auch bei der Photonentherapie ergibt.

Bei der Behandlungsplanung werden üblicherweise mehrere Bestrahlungsfelder mit verschiedenen Einfallswinkeln einzeln geplant. Jedes Bestrahlungsfeld ist auf das Scanning-System abgestimmt, d.h., bei der Planung wird jeweils ein Feld individuell geplant, dessen Ausmaß durch einen Scanbereich des Scanning-Systems begrenzt ist. Der Scanbereich ist durch die maximale Ablenkung des Partikelstrahls gegeben. Dabei unterscheidet man zwischen 2D-Scanning (die Ablenkung des Partikelstrahls erfolgt in zwei Richtung) und 1D-Scanning. Im 1D-Scanning wird zusätzlich der Patient schrittweise bewegt, um auch ein in der zweiten Dimension ausgedehntes Volumen bestrahlen zu können.

Problematisch wird die Bestrahlung eines Zielvolumens, das in der Bestrahlungsphase eine andere Lage- und/oder Größe aufweist, als sie in der Planungsphase der Erstellung des Bestrahlungsfeldes zugrunde lag. Denn aufgrund der Lage- und/oder Größenänderung des Zielvolumens liegt eventuell trotz erfolgter Positionsverifikation des Patienten das Zielvolumen nicht an der geplanten Stelle im Patienten.

Eine ähnliche Situation ergibt sich bei der Bestrahlung von sich z.B. aufgrund der Atmung bewegender Ziele. Diese Problematik wird beispielsweise in E. Rietzel et al., "Fourdimensional image based treatment planning: target volume segmentation and dose calculation in the presence of respiratory motion", Int. J. Radiation Oncology Biol. Phys. Vol. 61, No. 5 pp.1535-1550, 2005 behandelt. Darin und in den Zitatstellen werden des Weiteren Methoden der Segmentierung beschrieben, die für die Abgrenzung eines Tumorgewebes verwendet werden können. Allgemein sind Segmentierung und Registrierung von Bilddaten in der Radioonkologie bekannt.

Aufgaben der Erfindung sind es, die Durchführung einer Bestrahlung eines Volumens, dessen Lage und/oder Größe variiert, zu vereinfachen sowie eine entsprechende Strahlentherapieanlage anzugeben.

Diese Aufgaben werden erfindungsgemäß gelöst durch ein Verfahren nach Anspruch 1 und durch eine Strahlentherapieplanungsvorrichtung nach Anspruch 8.

Gemäß Anspruch 1 wird zur Anpassung eines Bestrahlungsfeldes für ein Zielvolumen, das seine Lage und/oder Form den Patienten mit der Zeit verändern kann, folgendermaßen vorgegangen. Ein üblicherweise bereits in einer Planungsphase der Strahlentherapie erzeugter 3D-Planungsbilddatensatz wird zusammen mit einem darauf geplanten Bestrahlungsfeld bereitgestellt. Das Bestrahlungsfeld entspricht einer Zuordnung von zu applizierenden Dosen zu einzelnen Volumenelementen des Zielvolumens. In der Behandlungsphase wird nun der Patient zur Bestrahlung vorbereitet und entsprechend positioniert. Aufgrund des späteren Zeitpunkts der Bestrahlungssitzung im Vergleich zur Bestrahlungsplanung kann das Zielvolumen seine Lage und/oder Form im Patienten verändert haben.

Um diese Veränderungen bei der Bestrahlung zu kompensieren, wird nun in der Bestrahlungsphase ein 3D-Bestrahlungssatz gewonnen. Dieser 3D-Bestrahlungsdatensatz wird mit dem 3D-Planungsbilddatensatz registriert, wobei eine zugehörige Transformation gewonnen wird, welche in einem übereinstimmenden Koordinatensystem die beispielsweise Verschiebung, Rotation, Skalierung, Scherung und/oder auch Deformation des Zielvolumens beschreibt. Um die Veränderung des Zielvolumens zu berücksichtigen, wird das Bestrahlungsfeld aus der Planungsphase mit dieser Transformation an die Bestrahlungsphase angepasst. Der 3D-Bestrahlungsdatensatz bildet dabei das zu bestrahlende Zielvolumen dreidimensional zum Zeitpunkt einer Bestrahlungssitzung, d.h. während einer Bestrahlungsphase, ab.

Dies hat den Vorteil, dass das geplante und für die Bestrahlung verifizierte und überprüfte Bestrahlungsfeld trotz der Änderung im Zielvolumen keine erneute Verifikation benötigt. Somit können kleine Lage- und/oder Formveränderungen des Zielvolumens schnell und einfach in der Bestrahlungsphase berücksichtigt werden. Bei größeren Veränderungen ist es von Vorteil, wenn zusätzlich die Dosen des Bestrahlungsfeldes anhand der Dichteverteilung im 3D-Bestrahlungsdatensatz im jeweiligen Eintrittskanal verifiziert oder angepasst werden. Somit kann beispielsweise die bei der Partikeltherapie erforderliche Genauigkeit in den Eindringtiefen der Teilchen gewährleistet werden.

Ferner kann sich beispielsweise bei der Bestrahlung mit einer Scanvorrichtung die Lage und/oder Formveränderung des Zielvolumens ein zusätzliches Regularisieren des Bestrahlungsfeldes als vorteilhaft erweisen. Dies ist beispielsweise dann der Fall, wenn einzelne Volumenelemente im Zielvolumen aufgrund einer Stauchung ihren Abstand ändern und so nicht mehr auf beispielsweise das Raster der Scanvorrichtung projiziert werden können. In einem solchen Fall mag es des Weiteren besonders vorteilhaft sein, dass die Dosen über das Bestrahlungsraster interpoliert werden. Somit kann auch bei stärkeren Lage- und/oder Formveränderungen des Zielvolumens der Bestrahlungsplan aufrechterhalten werden.

Die Registrierung kann manuell, teilautomatisiert oder automatisiert durchgeführt werden. Dabei markiert beispielsweise ein Bediener übereinstimmende Punkte in den verschiedenen 3D-Bilddatensätzen und lässt anhand dieser Eingabeparameter die Transformation der markierten Punkte berechnen. Eine Registrierung kann auch mithilfe von für das Zielvolumen signifikanten Hounsfield-Werten automatisiert durchgeführt werden.

Werden bei der Registrierung zusätzlich Bewegungen von Gewebe registriert, das das Zielvolumen umgibt, können auch diese Bewegungen zu einer Anpassung der Einstrahlrichtung genutzt werden. Diese Bewegungen können insbesondere dann Einfluss auf die Einstrahlrichtung haben, wenn dieses Gewebe nicht durchstrahlt werden darf. Auf diese Weise werden beispielsweise in der Nähe liegende zu schonende Gewebebereiche aus dem Eintrittskanal herausgehalten.

Wird bei der Registrierung festgestellt, dass die Lage- und/oder Formveränderung des Zielvolumens einen Grenzwert überschreiten, kann beispielsweise ein Warnsignal (z.B. akustisch oder visuell) erzeugt werden. Bewertet ein Bediener die Veränderungen als störend, kann er beispielsweise die Bestrahlungsphase abbrechen. Alternativ kann er Entwarnung für das Warnsignal geben und die Bestrahlung mit dem angepassten Bestrahlungsfeld veranlassen.

Die Aufgabe bezüglich der Strahlentherapieanlage wird gelöst in einem Aufbau gemäß Anspruch 8. Neben einer Strahlenquelle zum Erzeugen eines applizierbaren Bestrahlungsfeldes, das einstellbar ist, umfasst eine derartige Strahlentherapieanlage eine 3D-Bildgebungsvorrichtung zum Gewinnen eines 3D-Bestrahlungsdatensatzes in einer Bestrahlungsphase der Strahlentherapie. Ferner weist eine derartige Strahlentherapieanlage eine Anpasseinheit auf, die es erlaubt, das Bestrahlungsfeld auf eine in der Bestrahlungsphase vorliegende Lage- und/oder Formänderung anzupassen. Dazu ist die Anpasseinheit dazu ausgebildet, den in 3D-Bestrahlungsdaten mit einem bereitgestellten 3D-Planungsbilddatensatz zu registrieren und eine zugehörige Transformation zu gewinnen, welche die Lage- und/oder Formveränderung des Zielvolumens beschreibt. Ferner ist die Anpasseinheit dazu ausgebildet, ein bereit gestelltes Planungsfeld, welches in einer Planungsphase durch Zuordnung von zu applizierenden Dosen zu Volumenelementen des Zielvolumens anhand des 3D-Planungsbilddatensatzes geplant wird, mit der gewonnenen Transformation zu transformieren und an die Lage- und/oder Formveränderung des Zielvolumens in der Bestrahlungsphase anzupassen.

Eine derartige Strahlentherapieanlage erlaubt es, ohne erneute Verifikation eines Bestrahlungsfeldes dieses auch auf veränderte Zielvolumina anzuwenden. Dies beschleunigt die Strahlentherapie, da andernfalls benötigte Neuberechnungen von Bestrahlungsfeldern entfallen.

Die Strahlenquelle einer derartigen Strahlentherapieanlage kann zur Abgabe eines hochenergetischen Partikel- oder Photonenstrahls ausgebildet sein. Dieser ist vorzugsweise in seiner Energie, Abstrahlrichtung, Form, Rasterung, Energieverteilung und/oder Intensität einstellbar. Dies erlaubt es, die aufgrund der Transformation benötigten Änderungen der applizierten Strahlung vorzunehmen. Derartige Änderungen können z.B. auch dann vonnöten sein, wenn die Anpassungseinheit dazu ausgebildet ist, Dosen des Bestrahlungsfeldes anhand der Dichteverteilung im 3D-Bestrahlungsdatensatz im jeweiligen Eintrittskanal zu verifizieren oder anzupassen. Ferner können Einstellungsänderungen dann benötigt werden, wenn die Anpasseinheit dazu ausgebildet ist, das Bestrahlungsfeld aufgrund der Lage- und/oder Formveränderung zu regularisieren, insbesondere auf ein vorgegebenes Bestrahlungsraster zu interpolieren. Derartige Änderungen der applizierten Strahlung kann auch dann benötigt werden, wenn die Anpasseinheit ferner dazu ausgebildet ist, beispielsweise die Einstrahlrichtung an eine Lage- und/oder Formveränderung eines das Zielvolumen umgebenden Volumens anzupassen. Dadurch können beispielsweise Überdosierungen in zu schonendem Gewebe verhindert werden.

Weitere vorteilhafte Ausführungsformen der Strahlentherapieanlage und des Verfahrens sind durch die Merkmale der Unteransprüche gekennzeichnet.

Es folgt die Erläuterung von mehreren Ausführungsbeispielen der Erfindung anhand der Figuren 1 bis 4. Es zeigen:
- FIG 1: eine schematische Ansicht einer beispielhaften Partikeltherapieanlage,
- FIG 2: ein Ablauf-Diagramm zur Verdeutlichung einer Anpassung des Bestrahlungsfeldes,
- FIG 3: eine Skizze zur Verdeutlichung der Registrierung des Zielvolumens wie auch eines zu schonenden Gewebes in der Nähe des Einstrahlkanals, und
- FIG 4: eine Skizze zur Veränderung eines Bestrahlungsfeldes im Fall einer Translation und einer Deformation des Zielvolumens.

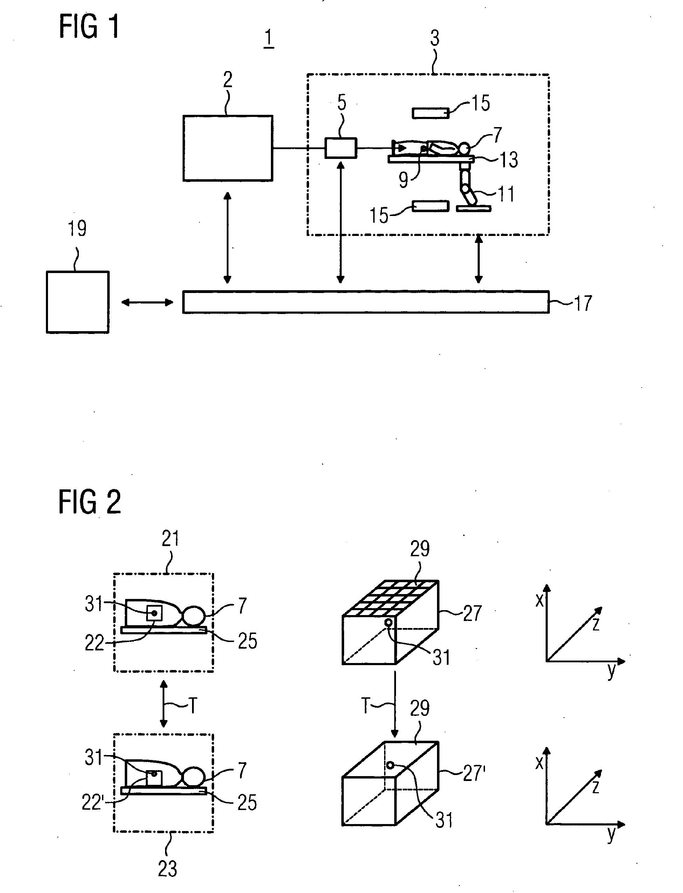

Figur 1 zeigt schematisch eine Strahlentherapieanlage 1 mit einer Strahlenquelle 2 und einem Bestrahlungsplatz 3. Man erkennt schematisch ferner ein Scansystem 5 mit einem dazu ausgerichteten Patienten 7. Die Strahlenquelle 2 weist im Fall einer Partikeltherapieanlage beispielsweise ein Beschleunigersystem (Synchrotron oder Zyklotron) und eine Hochenergiestrahlzuführung auf. Dort werden Partikel, d.h. insbesondere Ionen z.B. Protonen oder Kohlenstoffionen, auf Energien von bis zu einigen 100 MeV beschleunigt. Mit dem Scansystem 5 kann der Strahl vorzugsweise parallel innerhalb eines Scanbereich in seiner Strahllage eingestellt werden.

In Figur 1 soll beispielsweise eine Prostata des Patienten 7 bestrahlt werden. Die Lage der Prostata ändert sich täglich in Abhängigkeit von Blasen- und Darmfüllung. Die Prostata stellt ein Beispiel eines Zielvolumens 9 dar, das sich zeitabhängig in seiner Lage und/oder Größe mit der Zeit verändert. Gründe für derartige Änderungen liegen ferner auch im Rückgang oder im Wachstum von derartigen Tumoren sowie in den Änderungen des Gewichts und Formvariationen aufgrund des Füllungsgrades von benachbarten Organen. Auf einer kürzeren Zeitskala werden derartige Bewegungen auch durch die Peristaltik, dem Herzschlag oder die Atmung hervorgerufen. Bei entsprechend schneller Rechenleistung können auch derartige kurzzeitige Positionsänderungen in der Bestrahlung berücksichtigt werden.

Figur 1 zeigt ferner eine Positioniervorrichtung 11 (z.B. Roboter-basiert), welche eine flexible Einstellung der Ausrichtung der Patientenliege 13 ermöglicht. Somit können entweder Variationen der Bestrahlungsposition oder des Einfallswinkels a) patientenseitig mittels der Patientenpositioniervorrichtung 11 vorgenommen werden und/oder b) auf Seiten der Strahlzuführung mittels der Scanvorrichtung 5 und/oder durch eine Drehung einer Gantry.

Ferner erkennt man in Figur 1 schematisch eine 3D-Bildgebungsvorrichtung 15, die es erlaubt, in der hier verdeutlichten Bestrahlungsphase, d.h. kurz vor dem Auslösen der Applikation der Strahlung, eine 3D-Bildgebung des Zielvolumens und seiner Umgebung durchzuführen.

Ferner ist in Figur 1 eine Anpassvorrichtung 17 eingezeichnet, die beispielsweise in Form eines Kontrollsystems sowohl Einstellungen der Patientenpositioniervorrichtung 11 sowie der Scanvorrichtung 5, eines Gantrywinkels, Strahlenparameter der Strahlenquelle 2 (beispielsweise Energie, Energieverteilung, Intensität ...) veranlassen kann. Die Anpassvorrichtung 17 steht vorzugsweise in direktem Kontakt mit einem Arbeitsplatz 19 zur Therapieplanung, welcher beispielsweise ein Planungs-CT-Gerät und entsprechende Planungsprogramme zur Berechnung einer Dosisverteilung eines Bestrahlungsfeldes aufweist. Von dem Planungsarbeitsplatz 19 bezieht die Anpassvorrichtung 17 vorzugsweise direkt einen 3D-Planungsbilddatensatz mit zugehörigem Bestrahlungsfeld(ern). Die Anpassvorrichtung weist beispielsweise Ein- und Ausgabemittel auf, mit dem eine Registrierung des 3D-Planungsbilddatensatzes und des 3D-Strahlungsbilddatensatzes ausgelöst und überwacht werden kann. Eventuell kann die Anpassvorrichtung 17 diese Registrierung auch automatisiert vollziehen. Die mit der Registrierung des Zielvolumens verbundene Transformation steht der Anpassvorrichtung 17 für die Anpassung des Bestrahlungsfeldes auf eine Lage und/oder Formveränderung des Zielvolumens zur Verfügung. Die Anpassvorrichtung 17 verwendet die Transformation, um das Bestrahlungsfeld an die Veränderungen anzupassen.

Dieses Vorgehen verdeutlicht das Ablauf-Diagramm in Figur 2. Man erkennt den 3D-Planungsbilddatensatz 21 verdeutlicht durch einen schematischen Oberkörper des Patienten 7 mit dem Zielvolumen 22 in Form eines Rechtecks. Ferner erkennt man einen 3D-Bestrahlungsbilddatensatz 23, in dem das Zielvolumen 22 Richtung Patientenliege 25 verschoben dargestellt wird. Diese Verschiebung wird von der Anpassvorrichtung 17 durch die Transformation T bei der Registrierung der beiden 3D-Bilddatensätze bestimmt.

Ferner wird das dreidimensionale Bestrahlungsfeld 27 mit Volumenelementen 29 und den mittig in ihm liegenden Isozentrum 31 dargestellt. Die Geometrie des Bestrahlungsfeldes bei der Bestrahlung steht in geometrischen Bezug zum Isozentrum 31, welches sich üblicherweise im Zielvolumen 22 befindet. Die Anpassvorrichtung 17 lässt nun die Transformation T auf das Bestrahlungsfeld 27 wirken, so dass sich ein angepasstes Bestrahlungsfeld 27' ergibt, bei dem das Isozentrum 31 in X-Richtung verschoben ist und sich im oberen Bereich des Bestrahlungsfeldes befindet.

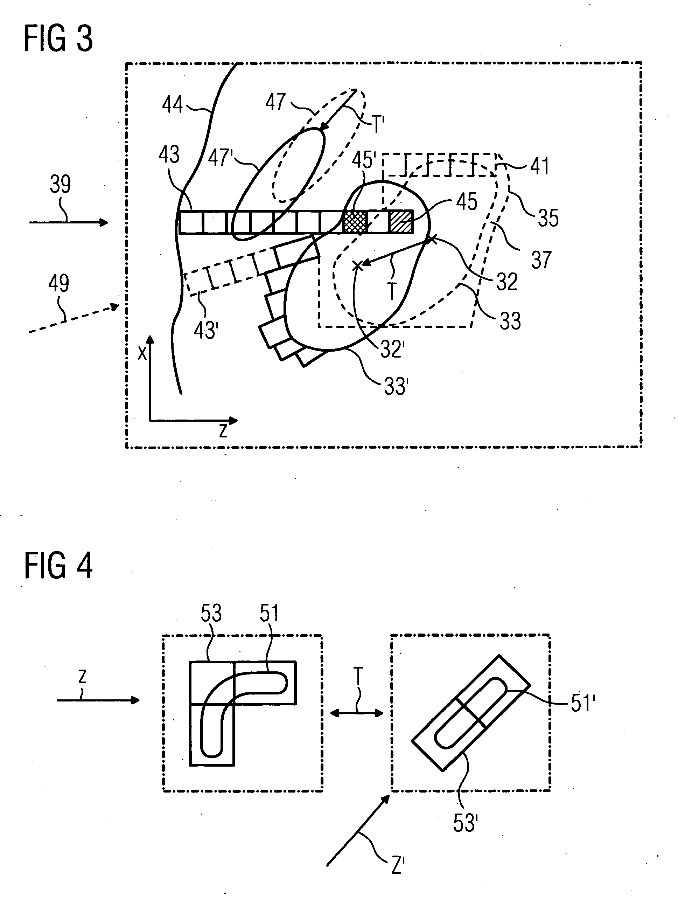

Figur 3 verdeutlicht anhand einer Skizze die möglichen Veränderungen im Fall einer beispielhaften Verschiebung eines Zielvolumens 33 (gestrichelt) auf ein verschobenes Zielvolumen 33' (durchgezogen) gemäß einer Transformation T. Eingezeichnet wurde für das Zielvolumen 33 ein Bestrahlungsfeld 35, das in seinen Ausmaßen nur einen geringfügigen Sicherheitskranz 37 aufweist, da die Lageveränderung aufgrund der vorgenommenen Anpassung des Bestrahlungsfeldes über die Transformation T berücksichtigt werden kann.

Würde keine Anpassung des Bestrahlungsfeldes vorgenommen werden, müsste das Bestrahlungsfeld 35 auch die Position des verschobenen Zielvolumens 33' abdecken, welches z.T. außerhalb des Sicherheitskranzes 37 liegt. Das Bestrahlungsfeld 35 kann bei der Bestrahlung mit einem Partikelstrahl in Z-Richtung als Ganzes bestrahlt werden oder bei Verwendung der Rasterscantechnik durch die Bestrahlung von einzelnen Volumenelementen 41 behandelt werden. Die an einem Volumenelement 41 zu applizierende Dosis wird im 3D-Planungs-Datensatz mithilfe der Schwächungskoeffizienten im Eintrittskanal berechnet. Schematisch ist ein Eintrittskanal 43 beginnend an der Körperoberfläche 44 für ein Volumenelement 45 eingezeichnet.

Vorerst soll nun eine zusätzliche aufgetretene Verschiebung eines verstärkt absorbierenden Gewebebereichs 47 vernachlässigt werden. Nach der Verschiebung T des Zielvolumens 33 ist der Eintrittskanal 43 für das Volumenelement 45' des verschobenen Zielvolumens 33' um zwei Volumenelemente verkürzt. Entsprechend ist die gleiche Einfallsrichtung im Partikelstrahl in seiner Energie zu reduzieren. Die benötigte Energie kann aufgrund der aus dem 3D-Bestrahlungsbilddatensatz berechneten Absorption des verkürzten Eintrittskanal 40 berechnet werden.

Die Verschiebung T zwischen den Isozentren 32 und 32' kann mithilfe der Registrierung der beiden Bilddatensätze gewonnen werden. Ist das stark absorbierende Gewebevolumen 47 nicht zu schonen, so kann die Einfallsrichtung in Z-Richtung bestehen bleiben.

Ist das Gewebevolumen 47 dagegen zu schonen und darf nicht im Eintrittskanal 43 liegen, kann durch zusätzliche Registrierung des Gewebevolumens 47 und der Berechnung der zugehörigen Transformation T' eine gedrehte Einfallsrichtung 49 für das Bestrahlungsfeld bestimmt werden. Aufgrund der Drehung ist bei Beibehaltung der Dosen in den einzelnen Volumenelementen des verschobenen Zielvolumens 33' eine neue Rasterung bzw. Formgebung des Partikelstrahls von nöten. In Figur 3 wird dies an den gekippten Volumenelementen verdeutlicht.

Figur 4 verdeutlicht die Anpassung des Bestrahlungsfeldes bei einer Formänderung. Im Planungsbilddatensatz wurde ein gebogenes Zielvolumen 51 mit einem aus drei Volumenelementen bestehenden Bestrahlungsfeld 53 zur Bestrahlung aus einer Richtung Z geplant. Im 3D-Bestrahlungsbilddatensatz erkennt man nun, dass sich das Zielvolumen gestreckt und zusammengezogen hat. Das geänderte Zielvolumen 51' kann nun mit zwei Volumenelementen eines Bestrahlungsfeldes 53' therapiert werden. Dabei wird die zuvor auf drei Volumenelemente verteilte Dosis nur mehr auf zwei Volumenelemente verteilt. Die Transformation T bewirkt ferner eine gedrehte Einstrahlrichtung Z'.

## Patentansprüche

1. Verfahren zur Anpassung eines Bestrahlungsfeldes für einen Bestrahlungsvorgang eines zu bestrahlenden Zielvolumens eines Patienten, das mit einem Partikelstrahl mit Hilfe der Scanning-Technik bestrahlt wird, bei der ein Partikelstrahl über das Zielvolumen punktweise gescannt wird,
wobei das Zielvolumen seine Lage und/oder Form im Patienten mit der Zeit verändern kann, mit folgenden Verfahrensmerkmalen:
- Bereitstellen eines 3D-Planungsbilddatensatzes und eines Bestrahlungsfeldes, welches in einer Planungsphase durch Zuordnen von nacheinander zu applizierenden Dosen zu Volumenelementen des Zielvolumens anhand des 3D-Planungsbilddatensatzes geplant wurden,
- Gewinnen eines 3D-Bestrahlungsdatensatzes in einer Bestrahlungsphase, welcher 3D-Bestrahlungsdatensatz das zu bestrahlende Zielvolumen abbildet,
- Registrieren des Zielvolumens im 3D-Planungsbilddatensatz und im 3D-Bestrahlungsdatensatz durch Gewinnen einer zugehörigen Transformation, welche eine Lage- und/oder Formveränderung des Zielvolumens im Patienten beschreibt,
- Anpassen des Bestrahlungsfeldes aus der Planungsphase mittels der Transformation an die Lage- und/oder Formveränderung des Zielvolumens in der Bestrahlungsphase, indem eine räumliche Lage der nacheinander zu den Volumenelementen zu applizierenden Dosen durch die Transformation transformiert wird.

2. Verfahren nach Anspruch 1, wobei zusätzlich die Dosen des Bestrahlungsfeldes anhand der Dichteverteilung im 3D-Bestrahlungsdatensatz im jeweiligen Eintrittskanal verifiziert oder angepasst werden.

3. Verfahren nach Anspruch 1 oder 2, wobei zusätzlich das Bestrahlungsfeldes aufgrund der Lage- und/oder Formveränderung des Zielvolumens regularisiert, insbesondere auf ein vorgegebenes Bestrahlungsraster interpoliert werden.

4. Verfahren nach einem der vorhergehenden Ansprüche, wobei die Registrierung manuell, teilautomatisiert oder automatisiert durchgeführt wird.

5. Verfahren nach einem der vorhergehenden Ansprüche, wobei als Transformation eine Translation, Rotation, Skalierung, Scherung und/oder Deformation bestimmt wird.

6. Verfahren nach einem der vorhergehenden Ansprüche, wobei zusätzlich die Einstrahlrichtung an eine Lage- und/oder Formveränderung eines das Zielvolumen umgebenden Volumens angepasst wird.

7. Verfahren nach einem der vorhergehenden Ansprüche, wobei bei einer einen Grenzwert überschreitenden Lage- und/oder Formveränderung des Zielvolumens ein Warnsignal erzeugt und eine Anpassung nur nach Entwarnung des Warnsignals durchgeführt wird.

8. Strahlentherapieanlage zum Bestrahlen eines zu bestrahlenden Zielvolumens eines Patienten, das mit einem Partikelstrahl mit Hilfe der Scanning-Technik bestrahlt wird, bei der ein Partikelstrahl über das Zielvolumen punktweise gescannt wird, wobei das Zielvolumen seine Lage und/oder Form im Patienten mit der Zeit verändern kann,
- mit einer Strahlenquelle, die zur Abgabe des Partikelstrahls ausgebildet ist und bei der ein applizierbares Bestrahlungsfeld einstellbar ist,
- mit einer 3D-Bildgebungsvorrichtung zum Gewinnen eines 3D-Bestrahlungsdatensatzes in einer Bestrahlungsphase und
- mit einer Anpasseinheit zur Anpassung des Bestrahlungsfeldes auf eine in der Bestrahlungsphase vorliegende Lage- und/oder Formveränderung, die dazu ausgebildet ist,
- den 3D-Bestrahlungsdatensatz mit einem bereitgestellten 3D-Planungsbilddatensatz zu registrieren und eine zugehörige Transformation zu gewinnen, welche die Lage- und/oder Formveränderung des Zielvolumens im Patienten beschreibt, und
- ein bereitgestelltes Bestrahlungsfeld, welches in einer Planungsphase durch Zuordnen von nacheinander zu applizierenden Dosen zu Volumenelementen des Zielvolumens anhand des 3D-Planungsbilddatensatzes geplant wird, mit der gewonnenen Transformation zu transformieren und an die Lage- und/oder Formveränderung des Zielvolumens in der Bestrahlungsphase anzupassen, indem eine räumliche Lage der nacheinander zu den Volumenelementen zu applizierenden Dosen durch die Transformation transformiert wird.

9. Strahlentherapieanlage nach Anspruch 8, wobei die Strahlenquelle zur Abgabe des hochenergetischen Partikelstrahls derart ausgebildet ist, dass der Partikelstrahl in seiner Energie und Abstrahlrichtung einstellbar ist.

10. Strahlentherapieanlage nach Anspruch 8 oder 9, wobei die 3D-Bildgebungsvorrichtung als Computertomograpiegerät oder als C-Arm-Röntgengerät ausgebildet ist.

11. Strahlentherapieanlage nach einem der Ansprüche 8 bis 10, wobei die Anpasseinheit ferner dazu ausgebildet ist, Dosen des Bestrahlungsfeldes anhand der Dichteverteilung im 3D-Bestrahlungsdatensatz im jeweiligen Eintrittskanal zu verifizieren oder anzupassen.

12. Strahlentherapieanlage nach einem der Ansprüche 8 bis 11, wobei die Anpasseinheit ferner dazu ausgebildet ist, das Bestrahlungsfeldes aufgrund der Lage- und/oder Formveränderung der Volumenelemente zu regularisieren, insbesondere auf ein vorgegebenes Bestrahlungsraster zu interpolieren.

13. Strahlentherapieanlage nach einem der Ansprüche 8 bis 12, wobei die Anpasseinheit ferner zur manuellen, teilautomatisierten oder automatisierten Registrierung ausgebildet ist.

14. Strahlentherapieanlage nach einem der Ansprüche 8 bis 13, wobei die Anpasseinheit ferner dazu ausgebildet ist, als Transformation eine Translation, Rotation, Skalierung, Scherung und/oder Deformation zu bestimmen.

15. Strahlentherapieanlage nach einem der Ansprüche 8 bis 14, wobei die Anpasseinheit ferner dazu ausgebildet ist, die Einstrahlrichtung an eine Lage- und/oder Formveränderung eines das Zielvolumen umgebenden Volumens anzupassen.

16. Strahlentherapieanlage nach einem der Ansprüche 8 bis 15, wobei die Anpasseinheit ferner dazu ausgebildet ist, bei einer einen Grenzwert überschreitenden Lage- und/oder Formveränderung des Zielvolumens ein Warnsignal zu erzeugen und eine Anpassung nur nach Entwarnung des Warnsignals durchzuführen.

## Claims

1. Method for adapting a radiation field for radiation treatment of an exposed target volume of a patient, which is radiated with a particle beam using scanning technology, wherein a particle beam is scanned point-by-point over the target volume,
wherein the target volume can change its position and/or shape in the patient over time, with the following method features:
- providing of a 3D planning image data set and a radiation field which in a planning phase was planned by associating doses that are to be consecutively applied with volume elements of the target volume on the basis of the 3D planning image data set,
- obtaining a 3D radiation data set in a radiation phase, said 3D radiation data set mapping the exposed target volume,
- registering the target volume in the 3D planning image data set and in the 3D radiation data set by obtaining an associated transformation which describes a change in position and/or shape of the target volume in the patient,
- adapting the radiation field from the planning phase by means of the transformation to the change in position and/or shape of the target volume in the radiation phase, in that a spatial position of the doses that are to be consecutively applied to the volume elements is transformed by the transformation.

2. Method according to claim 1, wherein additionally the doses of the radiation field are verified or adapted on the basis of the density distribution in the 3D radiation data set in the respective inlet channel.

3. Method according to claim 1 or 2, wherein additionally the radiation field is regularised on the basis of the change in position and/or shape of the target volume, and in particular is interpolated onto a predefined radiation grid.

4. Method according to one of the preceding claims, wherein the registration is performed manually, semi-automatically or automatically.

5. Method according to one of the preceding claims, wherein a translation, rotation, scaling, shearing and/or deformation is determined as a transformation.

6. Method according to one of the preceding claims, wherein additionally the beaming direction is adapted to a change in position and/or shape of a volume surrounding the target volume.

7. Method according to one of the preceding claims, wherein when a change in position and/or shape of the target volume exceeds a limit value, a warning signal is generated and an adaptation is performed only after the warning signal is given the all-clear.

8. Radiotherapy facility for radiating an exposed target volume of a patient, which is radiated with a particle beam using scanning technology, wherein a particle beam is scanned point-by-point over the target volume, wherein the target volume can change its position and/or shape in the patient over time,
- having a radiation source which is adapted to deliver the particle beam and for which an applicable radiation field can be set,
- having a 3D imaging apparatus for obtaining a 3D radiation data set in a radiation phase and
- having an adaptation unit to adapt the radiation field to a change in the position and/or shape occurring in the radiation phase, which is adapted to
- register the 3D radiation data set with a supplied 3D planning image data set and obtain an associated transformation which describes the change in position and/or shape of the target volume in the patient, and
- transform a supplied radiation field which is planned in a planning phase by associating doses that are to be consecutively applied with volume elements of the target volume on the basis of the 3D planning image data set, using the transformation obtained, and adapting said radiation field to the change in position and/or shape of the target volume in the radiation phase, in that a spatial position of the doses that are to be consecutively applied to the volume elements is transformed by the transformation.

9. Radiotherapy facility according to claim 8, wherein the radiation source is adapted to deliver the high-energy particle beam such that the particle beam can be adapted in terms of its energy and beam direction.

10. Radiotherapy facility according to claim 8 or 9, wherein the 3D imaging apparatus is designed as a computed tomography device or as a C-arm X-ray device.

11. Radiotherapy facility according to one of claims 8 to 10, wherein the adaptation unit is further adapted to verify or adapt doses of the radiation field on the basis of the density distribution in the 3D radiation data set in the respective inlet channel.

12. Radiotherapy facility according to one of claims 8 to 11, wherein the adaptation unit is further adapted to regularise the radiation field on the basis of the change in position and/or shape of the volume elements, in particular to interpolate to a predefined radiation grid.

13. Radiotherapy facility according to one of claims 8 to 12, wherein the adaptation unit is further designed for manual, semi-automatic or automatic registration.

14. Radiotherapy facility according to one of claims 8 to 13, wherein the adaptation unit is further adapted to determine a translation, rotation, scaling, shearing and/or deformation as a transformation.

15. Radiotherapy facility according to one of claims 8 to 14, wherein the adaptation unit is further adapted to adapt the beam direction to a change in position and/or shape of a volume surrounding the target volume.

16. Radiotherapy facility according to one of claims 8 to 15, wherein the adaptation unit is further adapted, when a change in position and/or shape of the target volume exceeds a limit value, to generate a warning signal and to perform an adaptation only after the warning signal is given the all-clear.

## Revendications

1. Procédé pour l'adaptation d'un champ d'irradiation pour un processus d'irradiation d'un volume cible d'un patient à irradier, le volume en question étant irradié avec un faisceau de particules à l'aide de la technique de balayage dans laquelle le volume cible est balayé point par point par un faisceau de particules,
la position et/ou la forme du volume cible pouvant être soumises à des modifications dans le patient au cours du temps, le procédé comprenant les caractéristiques opératoires ci-après :
- la procuration d'un jeu de données image d'une planification en 3D et d'un champ d'irradiation que l'on a planifié dans une phase de planification par attribution de doses à appliquer successivement à des éléments volumiques du volume cible en se référant au jeu de données image de la planification en 3D ;
- l'obtention d'un jeu de données d'une irradiation en 3D dans une phase d'irradiation, ledit jeu de données d'irradiation en 3D reproduisant le volume cible à irradier ;
- l'enregistrement du volume cible dans le jeu de données image de planification en 3D et dans le jeu de données d'irradiation en 3D par obtention d'une transformation correspondante qui décrit une modification de la position et/ou de la forme du volume cible dans le patient ;
- l'adaptation du champ d'irradiation provenant de la phase de planification au moyen de la transformation à la modification de la position et/ou de la forme du volume cible dans la phase d'irradiation, par le fait de transformer une position dans l'espace des doses à appliquer successivement aux éléments volumiques, via la transformation.

2. Procédé selon la revendication 1, dans lequel on vérifie ou on adapte en outre les doses du champ d'irradiation en se référant à la distribution de densité dans le jeu de données d'irradiation en 3D dans le canal d'entrée respectif.

3. Procédé selon la revendication 1 ou 2, dans lequel on procède en outre à une régularisation du champ d'irradiation sur base de la modification de la position et/ou de la forme du volume cible, en particulier en procédant à une interpolation sur une trame d'irradiation prédéfinie.

4. Procédé selon l'une quelconque des revendications précédentes, dans lequel on effectue l'enregistrement de manière manuelle, de manière partiellement automatisée ou de manière automatisée.

5. Procédé selon l'une quelconque des revendications précédentes, dans lequel on détermine comme transformation, une translation, une rotation, une mise à l'échelle, un cisaillement et/ou une déformation.

6. Procédé selon l'une quelconque des revendications précédentes, dans lequel on adapte en outre la direction d'irradiation à une modification de la position et/ou de la forme d'un volume entourant le volume cible.

7. Procédé selon l'une quelconque des revendications précédentes, dans lequel, dans le cas d'une modification de la position et/ou de la forme du volume cible qui dépasse une valeur limite, un signal d'avertissement est généré et une adaptation n'est mise en oeuvre qu'après la fin de l'alerte du signal d'avertissement.

8. Installation de radiothérapie pour l'irradiation d'un volume cible d'un patient à irradier, le volume en question étant irradié avec un faisceau de particules à l'aide de la technique de balayage dans laquelle le volume cible est balayé point par point par un faisceau de particules, la position et/ou la forme du volume cible pouvant être soumises à des modifications dans le patient au cours du temps, l'installation comprenant :
- une source d'irradiation qui est conçue pour délivrer le faisceau de particules et dans le cadre de laquelle on peut régler un champ d'irradiation applicable ;
- un dispositif de formation d'image en 3D pour l'obtention d'un jeu de données d'irradiation en 3D dans une phase d'irradiation ; et
- une unité d'adaptation pour adapter le champ d'irradiation à une modification de la position et/ou de la forme, présente dans la phase d'irradiation, ladite unité d'adaptation étant conçue
- pour enregistrer le jeu de données d'irradiation en 3D à un jeu de données image de la planification en 3D mis à disposition et pour obtenir une transformation correspondante qui décrit la modification de la position et/ou de la forme du volume cible dans le patient ; et
- pour transformer, avec la transformation obtenue, un champ d'irradiation mis à disposition qui est planifié dans une phase de planification par attribution de doses à appliquer successivement à des éléments volumiques du volume cible, en se référant au jeu de données image de la planification en 3D, et pour adapter le champ en question à la modification de la position et/ou de la forme du volume cible dans la phase d'irradiation par le fait de transformer une position dans l'espace des doses à appliquer successivement aux éléments volumiques, via la transformation.

9. Installation de radiothérapie selon la revendication 8, dans laquelle la source d'irradiation pour délivrer le faisceau de particules riches en énergie est conçue de telle sorte que le faisceau de particules peut être réglé en ce qui concerne son énergie et sa direction d'irradiation.

10. Installation de radiothérapie selon la revendication 8 ou 9, dans laquelle le dispositif de formation d'image en 3D est conçu sous la forme d'un appareil de tomodensitométrie ou sous la forme d'un appareil d'ampliphotographie sur arceau.

11. Installation de radiothérapie selon l'une quelconque des revendications 8 à 10, dans laquelle l'unité d'adaptation est en outre conçue pour vérifier ou pour adapter des doses du champ d'irradiation en se référant à la distribution de la densité dans le jeu de données d'irradiation en 3D, dans le canal d'entrée respectif.

12. Installation de radiothérapie selon l'une quelconque des revendications 8 à 11, dans laquelle l'unité d'adaptation est en outre conçue pour régulariser le champ d'irradiation sur base de la modification de la position et/ou de la forme des éléments volumiques, en particulier pour procéder à son interpolation sur une trame d'irradiation prédéfinie.

13. Installation de radiothérapie selon l'une quelconque des revendications 8 à 12, dans laquelle l'unité d'adaptation est en outre conçue pour l'enregistrement manuel, partiellement automatisé ou automatisé.

14. Installation de radiothérapie selon l'une quelconque des revendications 8 à 13, dans laquelle l'unité d'adaptation est en outre conçue pour déterminer comme transformation, une translation, une rotation, une mise à l'échelle, un cisaillement et/ou une déformation.

15. Installation de radiothérapie selon l'une quelconque des revendications 8 à 14, dans laquelle l'unité d'adaptation est en outre conçue pour adapter la direction d'irradiation à une modification de la position et/ou de la forme d'un volume entourant le volume cible.

16. Installation de radiothérapie selon l'une quelconque des revendications 8 à 15, dans laquelle l'unité d'adaptation est en outre conçue pour, dans le cas d'une modification de la position et/ou de la forme du volume cible qui dépasse une valeur limite, générer un signal d'avertissement et ne mettre en oeuvre une adaptation qu'après la fin de l'alerte du signal d'avertissement.
